# EUROPEAN PATENT APPLICATION

(11) **EP 1 583 017 A2**
(43) Date of publication of application: **05.10.2005**
(21) Application number: 05006282.7
(22) Date of filing: 22.03.2005
(51) Int. Cl.: G06F 19/00

(54) **Medical information management method and server and program using the same**

(30) Priority: 31.03.2004 JP 2004104948
(71) Applicant: Fuji Photo Film Co. Ltd., Kanagawa 250-0193 (JP)
(72) Inventor: Makino, Yoshinobu, Ashigarakami-gun, Kanagawa 258-8538 (JP)
(74) Representative: Klunker . Schmitt-Nilson . Hirsch

(57) **Abstract**

A medical information management method capable of preventing medical information from being browsed somewhere or the like when a client terminal is stolen or the like. A server (25) connected to a movable client terminal (40) via a network (11) includes a medical information recording unit (51), an access control information recording unit (52), a client terminal location acquiring unit (53), a record extractingunit (54), andamedical information access control unit (55).

## Description

### BACKGROUND OF THE INVENTION

### Field of the Invention

The present invention relates to a medical information management method of controlling accesses to medical information by movable client terminals, and further relates to a server and a program using such a medical information management method.

### Description of a Related Art

In medical institutions such as hospitals and clinics, LANs (Local Area Networks) have been provided and servers, client terminals, and various kinds of examination devices have been connected to the LANs. Further, notebook personal computers (hereinafter, also referred to as "notebook PCs") have been used as client terminals, and doctors, nurses, or the like have carried around the notebook PCs within the hospitals.

A possible problem when using the notebook PC is theft or loss of the notebook PC. When the notebook PC is thieved or lost, there is a possibility that personal information of patients is leaked. Accordingly, a technology of preventing the personal information of patients from leaking when the notebook PC is thieved or lost has been proposed.

MIYO Kengo, et al., "Development of Information Environment for Ubiquitous CPR", 23rd JCMI (Joint Conference on Medical Informatics), November, 2003, pp. 555-558 discloses that a host or server collectively manage all data in order not to leave clinical information in terminals, and further, all "my documents" directories are set to be redirected to the server in order not to leave the data created by users in terminals.

According to the technology, the clinical information or data created by users are never left in the terminals. However, if a person who stole the terminal connects the stolen terminal to any available outlet within the hospital, the stolen terminal is connected to a predetermined virtual LAN according to a MAC (Medial Access Control) address. Thereby, the person who stole the terminal can browse the clinical information that is managed by the host or server or data created by users.

### SUMMARY OF THE INVENTION

The present invention has been achieved in view of the above-described points. An object of the present invention is to provide a medical information management method capable of preventing medical information from being browsed somewhere when a client terminal is stolen. A further object of the present invention is to provide a medical information management server and a medical information management program using such a medical information management method.

In order to attain the above-described objects, a medical information management method according to the present invention is a method of managing medical information by using a server connected to a movable client terminal via a network, which method includes the steps of: (a) acquiring a location of the client terminal; (b) extracting a record corresponding to the location of the client terminal acquired at step (a) from a table having a plurality of records which store, in association with a plurality of locations, information for identifying medical information accessible when the client terminal exists in the respective locations; and (c) controlling access to the medical information by the client terminal based on the record extracted at step (b).

Further, a medical information management server according to the present invention is a server to be connected to amovable client terminal via a network, formanagingmedical information, which server includes: first recording means for recording medical information; second recording means for recording a table having a plurality of records which store, in association with a plurality of locations, information for identifying medical information accessible when the client terminal exists in the respective locations; first processing means for acquiring a location of the client terminal; second processing means for extracting from the table a record corresponding to the location of the client terminal acquired by the first processing means; and third processing means for controlling access to the medical information by the client terminal based on the record extracted by the second processing means.

Furthermore, a medical information management program according to the present invention is a program for managing medical information by using a server connected to a movable client terminal via a network, which program activates the server to execute the procedures of: (a) acquiring a location of the client terminal; (b) extracting a record corresponding to the location of the client terminal acquired at procedure (a) from a table having a plurality of records which store, in association with a plurality of locations, information for identifying medical information accessible when the client terminal exists in the respective locations; and (c) controlling access to the medical information by the client terminal based on the record extracted at procedure (b).

According to the present invention, accessible medical information can be changed according to the location of the client terminal. Thereby, when the client terminal is stolen, the medical information can be prevented from being browsed somewhere.

### BRIEF DESCRIPTION OF THE DRAWINGS

Fig. 1 is a block diagram showing a medical information management system using a medical information management method according to one embodiment of the present invention;
Fig. 2 is a block diagram showing a configuration of the medical information management server as shown in Fig. 1;
Fig. 3 shows an example of an access control information table recorded in an access control information recording unit as shown in Fig. 2;
Fig. 4 is a flowchart showing operation of the medical information management system as shown in Fig. 1;
Fig. 5 shows another example of the access control information table recorded in the access control information recording unit as shown in Fig. 2; and
Fig. 6 shows yet another example of the access control information table recorded in the access control information recording unit as shown in Fig. 2.

### DESCRIPTION OF THE PREFERRED EMBODIMENTS

Hereinafter, an embodiment of the present invention will be described in detail by referring to the drawings. The same reference numerals are assigned to the same component elements and the description thereof will be omitted.

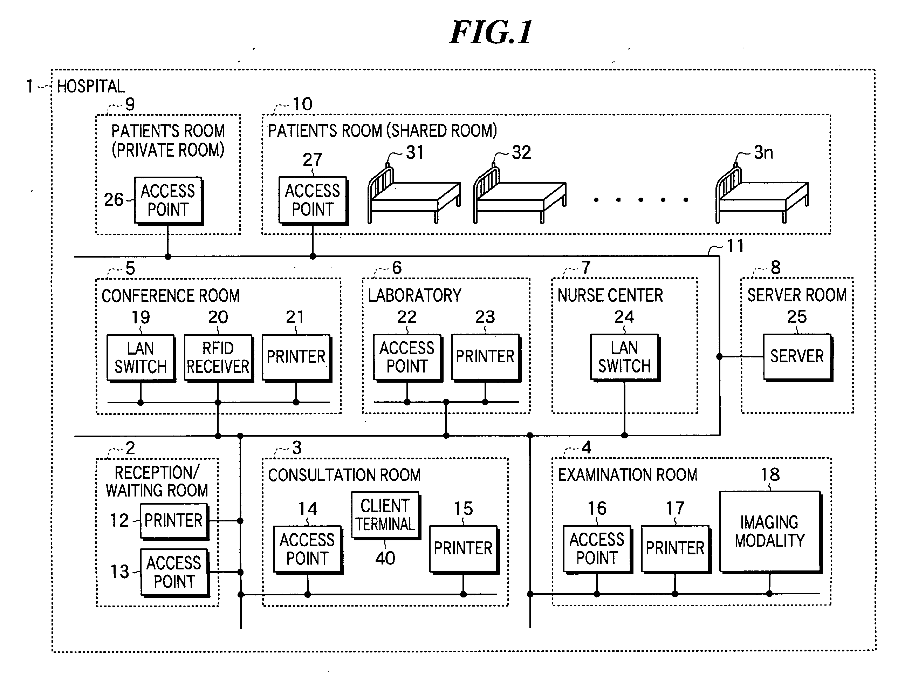

Fig. 1 is a block diagram showing a configuration of a medical information management system using a medical information management method according to one embodiment of the present invention. This medical information management system is installed in a hospital 1 as an example of medical facilities. The hospital 1 has a reception/waiting room 2, a consultation room 3, an examination room 4, a conference room 5, a laboratory 6, a nurse center 7, a server room 8, a patient's room (private room) 9, and a patient's room (shared room) 10, and a network 11 such as a LAN (Local Area Network) is provided between these rooms.

A printer 12 and an access point 13 for wireless LAN connection are provided in the reception/waiting room 2, and an access point 14 and a printer 15 are provided in the consultation room 3. Further, in the examination room 4, an access point 16, a printer 17, and an imaging modality 18 such as a radiation imaging device or an ultrasonic diagnostic device are provided. Further, in the conference room 5, a LAN switch 19 for wired LAN connection, anRFID (Radio Frequency ID) receiver 20 for detecting RFID tags, and a printer 21 are provided. Furthermore, an access point 22 and a printer 23 are provided in the laboratory 6, a LAN switch 24 is provided in the nurse center 7, a medical information management server 25 according to one embodiment of the present invention is provided in the server room 8, an access point 26 is provided in the patient's room (private room) 9, and an access point 27 is provided in the patient' s room (shared room) 10 . These devices are connected to the network 11.

Plural beds 31, 32 , ..., 3n are provided in the patient' s room (shared room) 10, and RFID tags, by which the beds can be uniquely identified, are respectively attached to the beds.

A portable client terminal 40 (e.g., a notebook PC or the like) is placed in the consultation room 3, and a doctor, nurse or the like performs medical services such as practice or nursing by carrying the client terminal 40 around in the hospital 1 and operating it. Although only one client terminal 40 is shown in Fig. 1, plural client terminals can be placed and used within the hospital 1. Further, although the client terminal 40 is carried within the hospital 1 in the embodiment, the client terminal 40 may be carried outside the hospital 1 for use in home nursing or home care.

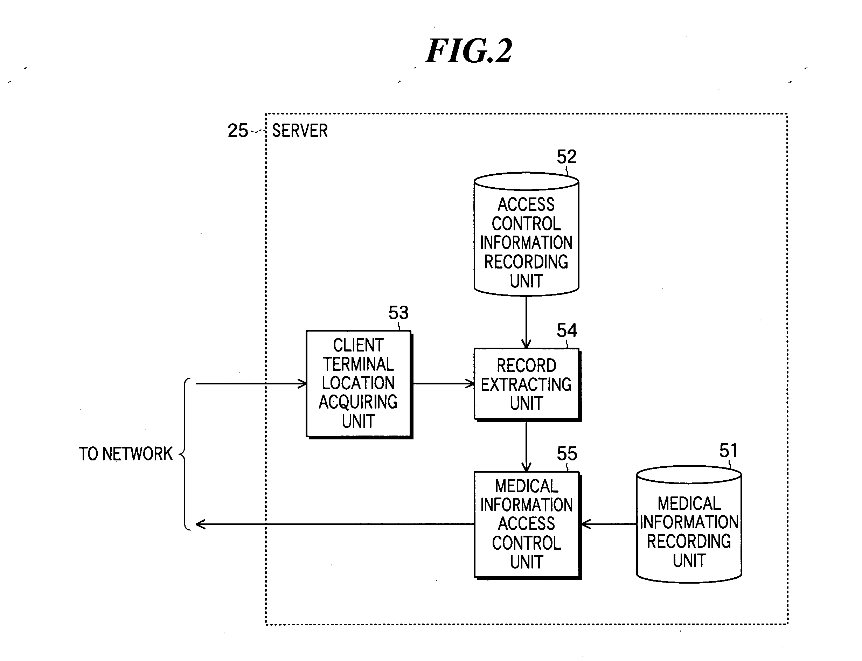

Fig. 2 is a block diagram showing a configuration of the medical information management server. As shown in Fig. 2, the medical information management server 25 includes a medical information recording unit 51, an access control information recording unit 52, a client terminal location acquiring unit 53, a record extracting unit 54, and a medical information access control unit 55.

The medical information recording unit 51 records various medical information such as electronic clinical chart database for storing electronic clinical charts which preserve information on diagnosis, prescription or the like with respect to each case, a medical image database for storing image data on medical images generated by the imaging modality 18 (Fig. 1), a visiting patient reception database for storing information on the visiting order of the visiting patients, consultation departments or the like, and so on.

The access control information recording unit 52 records an access control information table for identifying accessible medical information according to the location of the client terminal 40 within the hospital 1 among the medical information recorded in the medical information recording unit 51.

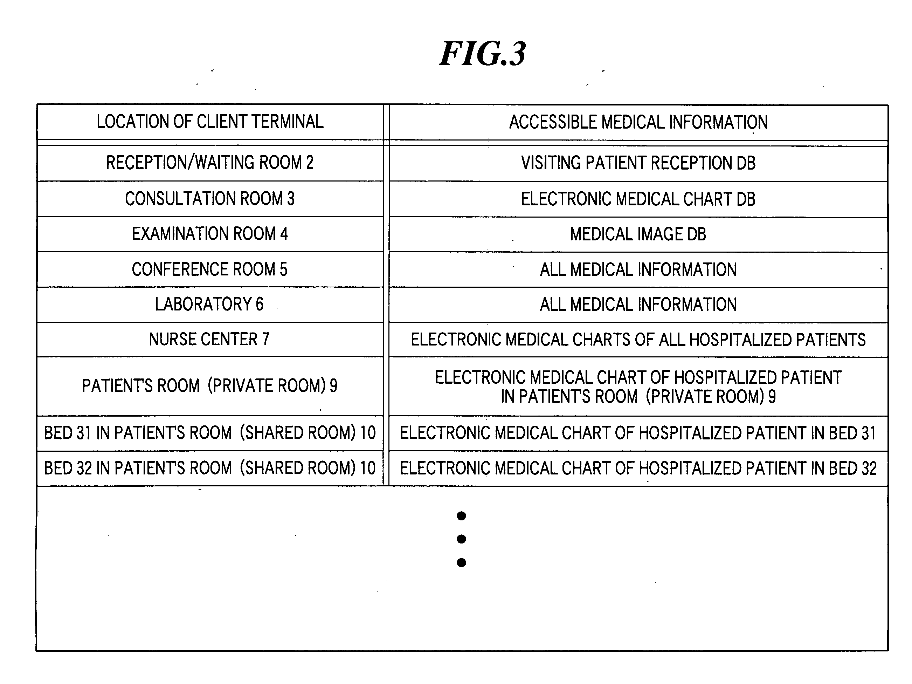

Fig. 3 shows an example of the access control information table recorded in the access control information recording unit 52. As shown in Fig. 3, the access control information table has a field for storing locations of the client terminal and a field for storing information for identifying medical information accessible when the client terminal exists in the respective locations.

For example, when the client terminal 40 is located in the reception/waiting room 2, the client terminal 40 is accessible to the visiting patient reception database (DB) . When the client terminal 40 is located in the consultation room 3, the client terminal 40 is accessible to the electronic clinical chart database (DB). When the client terminal 40 is located in the examination room 4, the client terminal 40 is accessible to the medical image database (DB).

Further, when the client terminal 40 is located in the conference room 5 or laboratory 6, the client terminal 40 is accessible to all medical information. When the client terminal 40 is located in the nurse center 7, the client terminal 40 is accessible to the clinical charts of all patients . When the client terminal 40 is located in the patient's room (private room) 9, the client terminal 40 is accessible to the clinical chart of the patient hospitalized in the patient' s room (private room) 9.

Furthermore, when the client terminal 40 is located at the bed 31 in the patient' s room (shared room) 10, the client terminal 40 is accessible to the clinical chart of the patient hospitalized in the bed 31 in the patient' s room (shared room) 10. When the client terminal 40 is located at the bed 32 in the patient's room (shared room) 10, the client terminal 40 is accessible to the clinical chart of the patient hospitalized in the bed 32 in the patient's room (shared room) 10.

By the way, in Fig. 3, in association with the locations of the client terminal, information for identifying accessible medical information when the client terminal exists in the respective locations is stored, however, in association with the locations of the client terminal and time or time period, information for identifying accessible medical information when the client terminal exists in the respective locations at that time or in that time period may be stored. For example, since the time period for the round of the patient's rooms is generally determined (e.g., in the afternoon or the like), if the access to the medical information from the patient' s room (private room) 9 and the patient's room (shared room) 10 can be made possible in that time period only, the security can be improved. Further, the access to the medical information from the examination room or a rehabilitation room may be made possible only in the examination time period or rehabilitation time period. Furthermore, the access to the medical information from outside of the hospital 1 may be made possible only in visiting time periods of the home nursing or home care.

When the access control information table as shown in Fig. 3 is generated, the location may be designated by displaying a floor plan of the hospital 1 on a display screen and selecting a desired room or the like on the floor plan. Further, by utilizing a shift management application program or the like, doctors, nurses and so on who can work at a desired time or in a desired time period may be displayed in a list so as to select a doctor or the like who is accessible to the medical information on the list. For example, a condition for the access to the medical information may be set as follows. In the case where doctor A goes the round on a certain day, only doctor A can access the medical information in the patient' s room (private room) 9 and the patient' s room (shared room) 10 during that day. Further, in the case where doctor B works the night shift on a certain day, doctor B is banned from accessing the medical information except for the emergency response during that day.

When the access control information table for the access to the medical information from the outside of the hospital 1 is generated for the home nursing, by utilizing a map display application program or navigation application program, the location may be designated by displaying a map image on the display screen and selecting a desired location on the map image. Further, by utilizing a shift management application program or the like, doctors, nurses and so on who can work at a desired time or in a desired time period is displayed in a list, and a doctor or the like who is accessible to medical information may be selected on the list.

Referring to Fig. 2 again, the client terminal location acquiring unit 53 acquires the location of the client terminal 40. The location of the client terminal 40 can be acquired by using an IP (Internet Protocol) address assigned to the client terminal 40, for example. As shown in Fig. 1, the client terminal 40 is connected to the network 11 via the access point 13 in the reception/waiting room 2, via the access point 14 in the consultation room 3, via the access point 16 in the examination room 4, via the LAN switch 19 in the conference room 5, via the access point 22 in the laboratory 6, via the LAN switch 24 in the nurse center 7, via the access point 26 in the patient's room (private room) 9 and via the access point 27 in the patient's room (shared room) 10. These access points or LAN switches assign IP addresses within respective predetermined ranges to the client terminal 40 by the DHCP (Dynamic Host Configuration Protocol) function, and therefore, the location of the client terminal 40 canbe acquiredaccording to the IP address assigned to the client terminal 40.

For example, the access point 13 is assigned an IP address within the range from "192.168.0.1" to "192.168.0.10", and the access point 14 is assigned an IP address within the range from "192.168.0.11" to "192.168.0.20". Thereby, the client terminal location acquiring unit 53 can acquire the location of the client terminal 40 in the reception/waiting room 2 when the IP address "192.168.0.5" is assigned to the client terminal 40.

By the way, an IP address may be assigned to the client terminal 40 by the medical information management server instead of the access point or LAN switch. Alternatively, a DHCP server may be separately provided.

Further, in the case where a predetermined RFID tag has been attached to the client terminal 40, the client terminal 40 can be identified as to be located within the conference room 5 by the RFID receiver 20. Although the RFID receiver 20 is provided only in the conference room 5 in Fig. 1, RFID receivers may be provided in the other rooms.

Furthermore, in the case where the RFID receiver has been mounted to the client terminal 40, the client terminal 40 detects that it is located at any one of the beds 31, 32, ... , 3n in the patient' s room (shared room) 10 for itself. Although the RFID tags are attached only to the beds 31, 32, ... , 3n in the patient's room (shared room) 10 in Fig. 1, RFID tags may be attached to the other rooms. Moreover, in place of the RFID tag, an entrance card, a paper on which a barcode is printed, and so on may be used.

Thus, the location of the client terminal 40 can be directly acquired by the medical information management server 25, or, after the client terminal 40 acquires the location of itself, it can inform the location to the medical information management server 25.

Referring to Fig. 2 again, the record extracting unit 54 extracts a record corresponding to the location of the client terminal 40 acquired by the client terminal location acquiring unit 53 from the access control information table. The medical information access control unit 55 performs access control to the medical information by the client terminal 40 based on the record extracted by the record extracting unit 54.

By the way, the client terminal location acquiring unit 53 , the record extracting unit 54, and the medical information access control unit 55 shown in Fig. 2 may be formed by a CPU and software (medical information management program) . The program, medical information, and access control information table can be recorded in a recording medium such as a hard disk, a flexible disk, an MO, an MT, a RAM, a CD-RW, or a DVD-RAM.

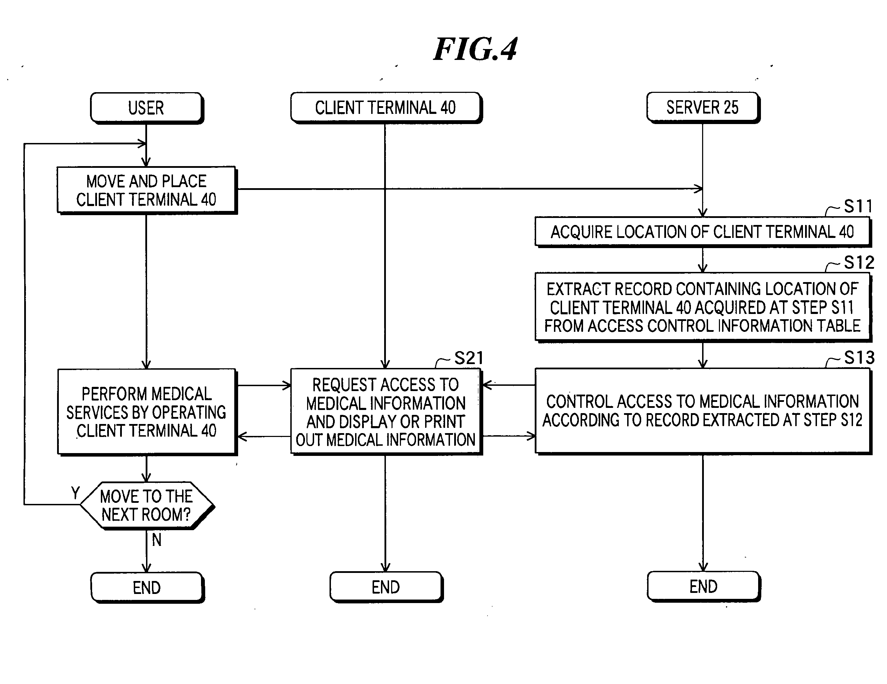

As below, the operation of the medical information management system will be described by referring to Figs. 1 to 4. Fig. 4 is a flowchart showing the operation of the medical information management system.

First, a user such as a doctor or nurse moves the client terminal 40 to a desired location and arranges it. In the case where a wired LAN is used, the user connects the client terminal 40 to the LAN switch by using a LAN cable. When a wireless LAN is used, negotiation is performed between the client terminal 40 and the access point.

When the client terminal 40 has been moved and arranged, the client terminal location acquiring unit 53 of the medical information management server 25 acquires the location of the client terminal 40 (step S11) . As described above, the location of the client terminal 40 may be acquired by using an IP address, an RFID tag, a barcode and so on. Further, the medical information management server 25 may directly acquire the location of the client terminal 40, or the client terminal 40 may detect the location of itself and inform the location to the medical information management server 25.

Then, the record extracting unit 54 of the medical information management server 25 extracts a record corresponding to the location of the client terminal 40 acquired at step S11 from the access control information table (step S12). For example, when the client terminal 40 is located in the consultation room 3, the record extracting unit 54 extracts the second record in the access control information table as shown in Fig. 3.

Then, the medical information access control unit 55 of the medical information management server 25 controls the access to the medical information by the client terminal 40 based on the record extracted by the record extracting unit 54 (step S13). Thereby, for example, in the case where the client terminal 40 is located in the consultation room 3, the client terminal 40 can access the electronic clinical chart data base. Here, the user such as a doctor operates the client terminal 40, and the client terminal 40 requests the access to the electronic clinical chart data base to the medical information management server 25 according to the operation of the user, and displays or prints out the content of the electronic clinical chart desired by the user (step S21). The user such as a doctor conducts medical services while watching the content of the electronic chart. By the way, the medical information that has been inputted to the client terminal 40 may be recorded in the medical information recording unit 51 within the medical information management server 25.

At this time, displayed items, input/selection items, the display screen design and so on may be changed according to the location of the client terminal 40. Further, some kind of program may be executed by the client terminal 40. Alternatively, the medical information management server 25 may execute some kind of program and cause the client terminal 40 to display the result.

Afterwards, when the user such as a doctor moves to another room, the user carries and arranges the client terminal 40. Thereby, the user can access medical information accessible in the other room.

Thus, accessible medical information can be changed according to the location of the client terminal 40. Thereby, for example, even when the client terminal 40 placed in the consultation room 3 is thieved by someone, the access to the electronic clinical chart database or the like canbeprevented in the reception/waiting room 2.

Further, when the client terminal 40 is located at the bed 31 of the patient's room (shared room) 10, the client terminal 40 can access only the electronic clinical chart of the patient in the bed 31 of the patient's room (shared room) 10. Thereby, when the doctor or the like looks away for several seconds, the patient in the bed 31 can be prevented from stealing a glance at the electronic clinical chart of the patient in the bed 32.

In the embodiment, the network 11 may be connected to a wide area network (e.g., Internet or the like) via a gateway having a firewall function. Further, the medical information management server 25 may be provided in an external data center or the like instead of the hospital 1, and connected to the network 11 via a wide area network. Furthermore, the access control to the medical information may be performed in a unit of a hospital, a building, a floor, a location in a room, a desk, a vicinity of a device and so on instead of a room.

Also, in the embodiment, a notebook personal computer is used as the client terminal 40, however, a tablet PC, PDA, desktop personal computer or the like may be used.

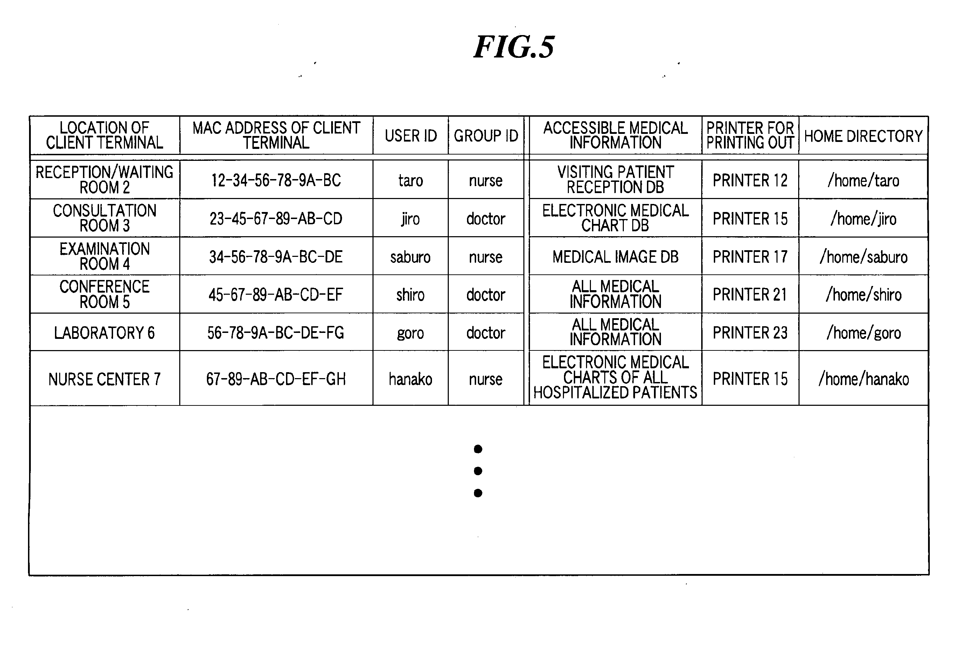

Further, in the access control information table, as shown in Fig. 5, information for identifying accessible medical information may be recorded according to the respective locations of the client terminal, respective MAC (Media Access Control) addresses of the client terminal, respective user IDs, and respective group IDs. Thereby, security can be improved. Here, in place of the MAC addresses, node names, host names or computer names of the client terminal may be used. For the purpose, the MAC address of the client terminal, the user ID, the group ID, or the node name, host name or computer name of the client terminal are acquired at step S11, and a record containing the information acquired at step S11 is extracted at step S12.

Furthermore, as shown in Fig. 5, in addition to the information for identifying accessible medical information, the operation environment may be set in association with the client terminal. In this example, printers for printing out and home directories have been set.

In Fig. 5, information for identifying accessible medical information is recorded in association with the respective locations of the client terminal, the respective MAC addresses of the client terminal, the respective user IDs and the respective group Ids. However, the information for identifying accessible medical information may be recorded in association with the respective locations of the client terminal and the respective MAC addresses of the client terminal, or the respective locations of the client terminal and the respective user IDs, or the respective locations of the client terminal and the respective group IDs, or the respective locations of the client terminal and the respective user IDs and the respective group IDs, or the like.

Alternatively, information for identifying accessible medical information may be stored in association with the respective locations of the client terminal, the respective MAC addresses of the client terminal, the respective user IDs, the respective group IDs and the respective times or time periods.

Further, a card reader is connected to the client terminal, and a personal ID card or the like of the doctor or the like may be read in place of the user IDs and the group IDs. Furthermore, as an emergency response, the medical information cannot be accessed unless plural IDs are inputted while the medical information can be accessed by inputting one user ID normally.

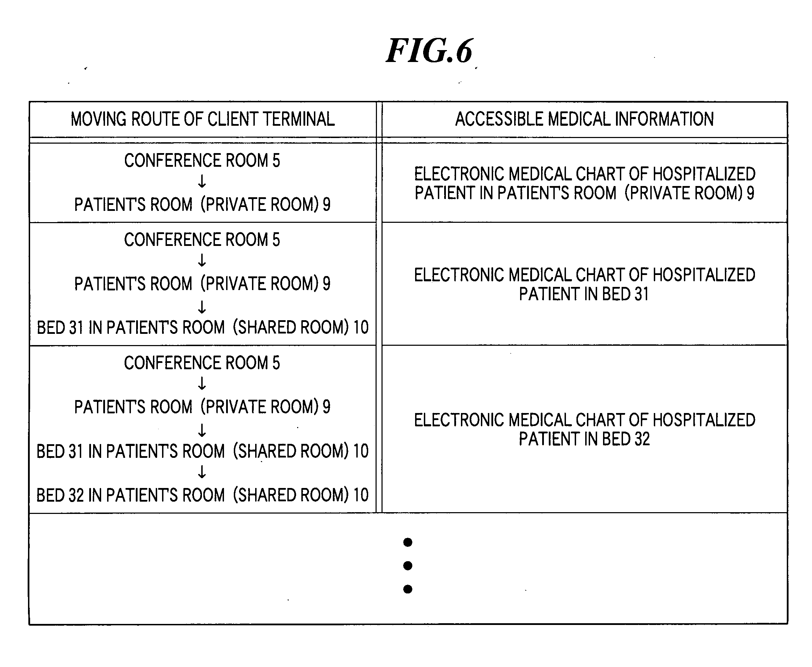

As amodification of the embodiment, in the access control information table, as shown in Fig. 6, information for identifying accessible medical information may be recorded according to the respective moving routes of the client terminal.

For example, when the client terminal moves from the conference room 5 to the patient's room (private room) 9, the client terminal is made accessible to the electronic clinical chart of the hospitalized patient in the patient' s room (private room) 9 (step S13) . Further, by predicting that the client terminal then moves to the bed 31 in the patient' s room (shared room) 10, preparation may be made to transmit the electronic clinical chart of the hospitalized patient in the bed 31 in the patient's room (shared room) 10 to the client terminal. As the preparation, for example, the electronic clinical chart of the hospitalized patient in the bed 31 may be read from a hard disk drive and buffered in a transmission buffer, or the electronic clinical chart of the hospitalized patient in the bed 31 may be transmitted to a non-rendered area of a VRAM (Video RAM) of the client terminal. Thereby, the access to the medical information can be made faster.

In the modification of the embodiment, the access to the medical information may be controlled according to combinations of the respective moving routes of the client terminal, the respective MAC addresses of the client terminal, the respective user IDs and/or the respective group IDs. Further, when the client terminal is located within the examination room 4, the client terminal may be allowed to execute a console program for operating the imaging modality. Furthermore, one client terminal may function as a server for providing data to other client terminals and/or the medical information management server 25.

## Claims

1. A method of managing medical information by using a server (25) connected to a movable client terminal (40) via a network (11), said method comprising the steps of:
(a) acquiring a location of said client terminal (40);
(b) extracting a record corresponding to the location of said client terminal (40) acquired at step (a) from a table having a plurality of records which store, in association with a plurality of locations, information for identifying medical information accessible when said client terminal (40) exists in the respective locations; and
(c) controlling access to the medical information by said client terminal (40) based on the record extracted at step (b).

2. The method according to claim 1, wherein step (a) includes acquiring the location of said client terminal (40) by using one of a network address assigned to said client terminal (40) and an RFID (Radio Frequency ID) tag and/or a barcode placed in said client terminal (40) and/or a medical institution.

3. The method according to claim 1, wherein:
said table has a plurality of records which store, in association with a plurality of moving routes, information for identifying medical information accessible when said client terminal (40) moves along the respective moving routes; and
step (b) includes extracting a record corresponding to a moving route along which said client terminal (40) has moved.

4. The method according to claim 3, wherein step (c) includes predicting a next moving destination of said client terminal (40) based on the record extracted at step (b), and preparing for access to medical information accessible when said client terminal (40) moves to the next moving destination.

5. The method according to claim 1, wherein:
said table has a plurality of records which store information for identifying medical information accessible by said client terminal (40) in association with identifiers for identifying said client terminal (40), identifiers for identifying users using said client terminal (40) and/or identifiers for identifying groups to which users using said client terminal (40) belongs in addition to a plurality of locations and/or a plurality of moving routes;
step (a) includes acquiring an identifier for identifying said client terminal (40), an identifier for identifying a user using said client terminal (40) and/or an identifier for identifying a group to which the user using said client terminal (40) belongs in addition to a location and/or a moving route of said client terminal (40); and
step (b) includes extracting from said table a record corresponding to the identifier for identifying said client terminal (40), the identifier for identifying the user using said client terminal (40) and/or the identifier for identifying the group to which the user using said client terminal (40) belongs in addition to the location and/or the moving route of said client terminal (40) acquired at step (a) .

6. The method according to claim 5, wherein:
said table has a plurality of records which store information for identifying medical information accessible by said client terminal (40) in association with identifiers for identifying said client terminal (40), identifiers for identifying users using said client terminal (40) and/or identifiers for identifying groups to which users using said client terminal (40) belong in addition to a plurality of locations and/or a plurality of moving routes and times and/or time periods;
step (a) includes acquiring an identifier for identifying said client terminal (40), an identifier for identifying a user using said client terminal (40) and/or an identifier for identifying a group to which the user using said client terminal (40) belongs in addition to a location and/or a moving route of said client terminal (40) and a time and/or a time period; and
step (b) includes extracting from said table a record corresponding to the identifier for identifying said client terminal (40), the identifier for identifying the user using said client terminal (40) and/or the identifier for identifying the group to which the user using said client terminal (40) belongs in addition to the location and/or the moving route of said client terminal (40) and the time and/or the time period acquired at step (a).

7. The method according to claim 1, wherein step (c) includes controlling said client terminal (40) to set an operation environment and/or execute a predetermined program.

8. A server (25) to be connected to a movable client terminal (40) via a network (11), for managing medical information, said server (25) comprising:
first recording means (51) for recording medical information;
second recording means (52) for recording a table having a plurality of records which store, in association with a plurality of locations, information for identifying medical information accessible when said client terminal (40) exists in the respective locations;
first processing means (53) for acquiring a location of said client terminal (40);
second processing means (54) for extracting from said table a record corresponding to the location of said client terminal (40) acquired by said first processing means (53) ; and
third processing means (55) for controlling access to the medical information by said client terminal (40) based on the record extracted by said second processing means (54) .

9. The server (25) according to claim 8, wherein said first processing means (53) acquires the location of said client terminal (40) by using one of a network address assigned to said client terminal (40) and an RFID (Radio Frequency ID) tag and/or a barcode placed in said client terminal (40) and/or a medical institution (1).

10. The server (25) according to claim 8, wherein:
said table has a plurality of records which store, in association with a plurality of moving routes, information for identifying medical information accessible when said client terminal (40) moves along the respective moving routes; and
said second processing means (54) extracts a record corresponding to a moving route along which said client terminal (40) has moved.

11. The server (25) according to claim 10, wherein said third processing means (55) predicts a next moving destination of said client terminal (40) based on the record extracted by said second processing means (54), and preparing for access to the medical information accessible when said client terminal (40) moves to the next moving destination.

12. The server (25) according to claim 8, wherein:
said table has a plurality of records which store information for identifying medical information accessible by said client terminal (40) in association with identifiers for identifying said client terminal (40), identifiers for identifying users using said client terminal (40) and/or identifiers for identifying groups to which users using said client terminal (40) belong in addition to a plurality of locations and/or a plurality of moving routes;
said first processing means (53) acquires an identifier for identifying said client terminal (40), an identifier for identifying a user using said client terminal (40) and/or an identifier for identifying a group to which the user using said client terminal (40) belongs in addition to a location and/or a moving route of said client terminal (40); and
said second processing means (54) extracts from said table a record corresponding to the identifier for identifying said client terminal (40), the identifier for identifying the user using said client terminal (40) and/or the identifier for identifying the group to which the user using said client terminal (40) belongs in addition to the location and/or the moving route of said client terminal (40) acquired by said first processing means (53).

13. The server (25) according to claim 12, wherein:
said table has a plurality of records which store information for identifying medical information accessible by said client terminal (40) in association with identifiers for identifying said client terminal (40), identifiers for identifying users using said client terminal (40) and/or identifiers for identifying groups to which users using said client terminal (40) belong in addition to a plurality of locations and/or a plurality of moving routes and times and/or time periods;
said first processing means (53) acquires an identifier for identifying said client terminal (40), an identifier for identifying a user using said client terminal (40) and/or an identifier for identifying a group to which the user using said client terminal (40) belongs in addition to a location and/or a moving route of said client terminal (40) and a time and/or a time period; and
said second processing means (54) extracts from said table a record corresponding to the identifier for identifying said client terminal (40), the identifier for identifying the user using said client terminal (40) and/or the identifier for identifying the group to which the user using said client terminal (40) belongs in addition to the location and/or the moving route of said client terminal (40) and the time and/or the time period acquired by said first processing means (53) .

14. The method according to claim 8, wherein said third processing means (55) controls access to said medical information by said client terminal (40) and controls said client terminal (40) to set an operation environment and/or execute a predetermined program.

15. A program for managing medical information by using a server (25) connected to a movable client terminal (40) via a network (11), said program activating said server (25) to execute the procedures of:
(a) acquiring a location of said client terminal (40);
(b) extracting a record corresponding to the location of said client terminal (40) acquired at procedure (a) from a table having a plurality of records which store, in association with a plurality of locations, information for identifying medical information accessible when said client terminal (40) exists in the respective locations; and
(c) controlling access to the medical information by said client terminal (40) based on the record extracted at procedure (b).

16. The program according to claim 15, wherein procedure (a) includes acquiring the location of said client terminal (40) by using one of a network address assigned to said client terminal (40) and an RFID (Radio Frequency ID) tag and/or a barcode placed in said client terminal (40) and/or a medical institution (1).

17. The program according to claim 15, wherein said table has a plurality of records which store, in association with a plurality of moving routes, information for identifying medical information accessible when said client terminal (40) moves along the respective moving routes; and
procedure (b) includes extracting a record corresponding to a moving route along which said client terminal (40) has moved.

18. The program according to claim 17, wherein procedure (c) includes predicting a next moving destination of said client terminal (40) based on the record extracted at procedure (b), and preparing for access to the medical information accessible when said client terminal (40) moves to the next moving destination.

19. The program according to claim 15, wherein:
said table has a plurality of records which store information for identifying medical information accessible by said client terminal (40) in association with identifiers for identifying said client terminal (40), identifiers for identifying users using said client terminal (40) and/or identifiers for identifying groups to which users using said client terminal (40) belong in addition to a plurality of locations and/or a plurality of moving routes;
procedure (a) includes acquiring an identifier for identifying said client terminal (40), an identifier for identifying a user using said client terminal (40) and/or an identifier for identifying a group to which the user using said client terminal (40) belongs in addition to a location and/or a moving route of said client terminal (40); and
procedure (b) includes extracting from said table a record corresponding to the identifier for identifying said client terminal (40), the identifier for identifying the user using said client terminal (40) and/or the identifier for identifying the group to which the user using said client terminal (40) belongs in addition to the location and/or the moving route of said client terminal (40) acquired at procedure (a) .

20. The program according to claim 19, wherein:
said table has a plurality of records which store information for identifying medical information accessible by said client terminal (40) in association with identifiers for identifying said client terminal (40), identifiers for identifying users using said client terminal (40) and/or identifiers for identifying groups to which users using said client terminal (40) belong in addition to a plurality of locations and/or a plurality of moving routes and times and/or time periods;
procedure (a) includes acquiring an identifier for identifying said client terminal (40), an identifier for identifying a user using said client terminal (40) and/or an identifier for identifying a group to which the user using said client terminal (40) belongs in addition to a location and/or a moving route of said client terminal (40) and a time and/or a time period; and
procedure (b) includes extracting from said table a record corresponding to the identifier for identifying said client terminal (40), the identifier for identifying the user using said client terminal (40) and/or the identifier for identifying the group to which the user using said client terminal (40) belongs in addition to the location and/or the moving route of said client terminal (40) and the time and/or the time period acquired at procedure (a).

21. The program according to claim 15, wherein procedure (c) includes controlling said client terminal (40) to set an operation environment and/or execute a predetermined program.
